(19) Europäisches Patentamt
European Patent Office
Office européen des brevets

(11) Publication number: **0 392 796 A2**

# (12) EUROPEAN PATENT APPLICATION

(21) Application number: **90303829.7**

(51) Int. Cl.5: **C07D 501/24, A61K 31/545**

(22) Date of filing: **10.04.90**

(30) Priority: **12.04.89 JP 92615/89**

(43) Date of publication of application:
**17.10.90 Bulletin 90/42**

(84) Designated Contracting States:
**AT BE CH DE DK ES FR GB GR IT LI LU NL SE**

(71) Applicant: **YAMANOUCHI PHARMACEUTICAL CO. LTD.**
**No. 3-11 Nihonbashi-Honcho, 2-chome**
**Chuo-ku**
**Tokyo(JP)**

(72) Inventor: **Nagano, Noriaki**
**2240-77, Wakashiba-cho**
**Ryugasaki-shi, Ibaraki(JP)**
Inventor: **Satch, Masato**
**Roomy Tsukuba 321, 5-9, Ninomiya 2-chome**
**Tsukuba-shi, Ibaraki(JP)**
Inventor: **Komiya, Masauki**
**2-1, Nishibori 9-chome**
**Urawa-shi, Saitama(JP)**
Inventor: **Okazaki, Toshio**
**Etowahru Kasuga 203, 35-2, Kasuga 2-chome**
**Tusukuba-shi, Ibaraki(JP)**
Inventor: **Maeda, Tetsuya**
**14-6, Yotsuya 3-chome**
**Urawa-shi, Saitama(JP)**
Inventor: **Shibanuma, Tadao**
**596-11, Ohyaguchi**
**Urawa-shi, Saitama(JP)**

(74) Representative: **Geering, Keith Edwin et al**
**REDDIE & GROSE 16 Theobalds Road**
**London WC1X 8PL(GB)**

## (54) Cephalosporin derivatives.

(57) A 7-[2-(2-amino-4-thiazolyl)-2-(hydroxyimino)acetamido]-3-(1,3-dithiolan-2-yl]-3-cephem-4-carboxylic acid derivative of formula 1 or a salt thereof

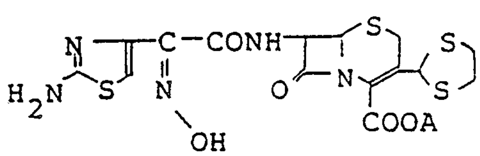

(I)

wherein A represents a hydrogen atom, a 1-acetoxyethyl group, a 1-cyclohexylacetoxyethyl group or a pivaloyloxymethyl group. These compounds are effective antimicrobial agents suitable for oral administration.

EP 0 392 796 A2

## CEPHALOSPORIN DERIVATIVES

The present invention relates to cephalosporin type antimicrobial compounds showing good oral absorption.

The compounds of the present invention are 7-[2-(2-amino-4-thiazolyl)-2-(hydroxyimino)acetamido]-3-(1,3-dithiolan-2-yl)-3-cephem-4-carboxylic acid derivatives (I) and salts thereof:

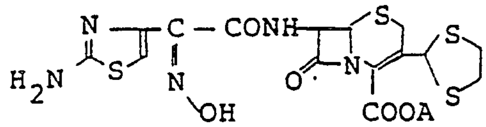

wherein A represents a hydrogen atom, a 1-acetoxyethyl group, a 1-cyclohexylacetoxyethyl group or a pivaloyloxymethyl group.

Said groups A have the formulae

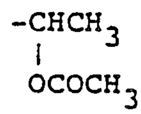 , 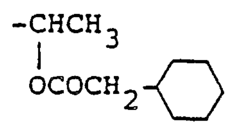

and $-CH_2OCOC(CH_3)_3$ respectively.

The compounds provided by the present invention are novel cephalosporin derivatives having the chemical structural characteristics of the 1,3-dithiolan-2-yl group at the 3-position of the cephalosporin nucleus and the hydroxyimino group substituted at the α-position of the acetamido group at the 7-postion.

These compounds have good absorption rate from the digestive tract and exhibit high antimicrobial activity and are therefore suitable for oral administration. The antimicrobial activity of the compound (I) where A is hydrogen (i.e. 7-[2-(2-amino-4-thiazolyl)-2-(hydroxyimino)acetamido]-3-(1,3-dithiolan-2-yl)-3-cephem-4-carboxylic acid) is shown in Table 1.

Table 1

| (Minimum Growth Inhibition Concentration, γ/ml) | |
|---|---|
| Name of Strain | Antimicrobial Activity |
| Staphylococcus aureus FDA 209P JC-1 | 0.39 |
| Staphylococcus epidermidis IID 866 | 0.39 |
| Enterobacter Faecalis CAY 1104 | 3.13 |
| Escherichia coli 0-1 | 0.39 |
| Klebsiella pneumoniae ATCC 10031 | 0.78 |

The absorption rate from the digestive tract of the compound (I) wherein A is the 1-acetoxyethyl group is shown below.

Ether-anesthetized rats were fixed belly upwards and polyethylene tubes inserted into the bile-ducts. Urine was collected from the same rats. After the test compound was orally administered at 50 mg/kg, urine and bile samples were collected within 0 - 6 hours and also within 6 -24 hours. The amounts recovered in the urine and the bile within 24 hours were determined by measuring the test compound concentrations in the urine and the bile by a bioassay method using a standard solution of the test compound prepared with M/15 phosphate buffer.

| (2) Test Results (Absorption Rate) | | | |
|---|---|---|---|
| | Recovery Rate (%) within 24 hours | | |
| | In the Urine | In the Bile | Total |
| Test Compound | 14.1 | 8.5 | 22.6 |

The compounds (I) of the present invention may be administered as such, or as salts with pharmacologically acceptable acids or bases - e.g inorganic acid salts such as hydrohalogenic acid salts (hydrochloric acid salts, hydrobromic acid salts etc.), sulfates etc; salts with organic acids such as formates, acetates, fumarates, citrates etc; alkali metal salts; and salts with organic bases and basic amino acids such as dicyclohexylamine, triethylamine, arginine, lysine etc.

The compounds of the present invention are mainly orally administered as tablets, capsules, syrups etc. The dosage varies depending on the graveness of the condition, the body weight etc., but suitably is 200 - 3,000mg per day for adults, administered in 1 or 2 portions. The preparation of the respective formulations may be effected in conventional manner using excipients, preservatives, stabilizers etc. employed in the pharmaceutical field.

The compounds of the present invention may be produced by the following reaction schemes:

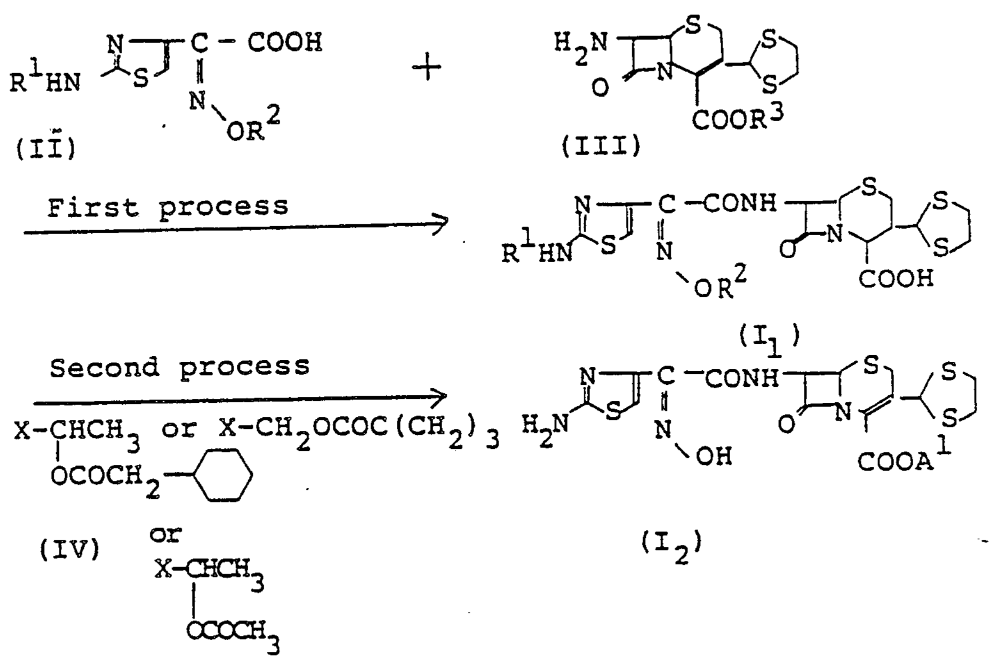


wherein $A^1$ represents a 1-acetoxyethyl group, a 1-cyclohexylacetoxyethyl group or a pivaloyloxymethyl group, $R^1$ represents a hydrogen atom or a protective group for the amino group, $R^2$ represents a hydrogen atom or a protective group for the hydroxyl group, $R^3$ represents a hydrogen atom or a protective group for the carboxyl group, and X represents a halogen atom.

The first step comprises the reaction (amidation) of alkoxyiminothiazole acetic acid derivative (II) or reactive derivative thereof at thc carboxyl group with 7-amino-3-(1,3-dithiolan- 2-yl)cephalosporin derivative (III) and thereafter the removal if necessary of any protective group(s) for the carboxyl, amino and hydroxyl groups in the product.

As protective groups for the carboxyl group, there may be specifically mentioned protective groups which may be removed easily under mild conditions, such as trimethylsilyl, benzhydryl, β-methylsulfonylethyl, phenacyl, p-methoxybenzyl, tert-butyl and p-nitrobenzyl groups etc.

As protective groups for the amino group, there may be mentioned, for example, acyl groups such as formyl, acetyl, propionyl, tertiary (abbreviated as tert or t)-butoxycarbonyl, methoxyacetyl, methoxypropionyl, benzyloxycarbonyl, and p-nitrobenzyloxycarbonyl groups etc.; aralkyl groups such as benzyl, benzhydryl, and trityl groups etc.;and the like.

As protective groups for the hydroxyl group, there are e.g. a (1-methoxy-1-methyl)ethyl group and lower alkylsilyl groups such as trimethylsilyl etc., and the like.

The amidation reaction is generally conducted in a solvent with cooling or at room temperature. The solvent is not particularly restricted as long as it does not participate in the reaction, but as those generally employed, there may be mentioned organic solvents such as dioxane, tetrahydrofuran, ether, acetone, methyl ethyl ketone, chloroform, methylene chloride, ethylene chloride, methanol, ethanol, acetonitrile, ethyl acetate, ethyl formate, dimethylformamide, dimethylsulfoxide etc. These solvents may be used alone or in appropriate mixtures.

Compound (II) may be used in the form of free carboxylic acid or reactive carboxylic acid derivative; suitable such derivatives include mixed acid anhydrides, acid halides, activated esters, activated amides, acid anhydrides, acid azides etc. When compound (II) is a free carboxylic acid, it is preferred to use a condensing agent such as N,N′-dicyclohexylcarbodiimide, N,N′-diethylcarbodiimide etc.

With some reactive carboxylic acid derivatives it is preferred for smooth operation to react in the presence of a base - e.g. there may be mentioned inorganic bases such as sodium and potassium bicarbonates and carbonates etc., and organic bases such as trimethylamine, triethylamine, dimethylaniline, pyridine etc.

The removal of the protective group from the thus obtained product may be easily effected, for example, by using acid in the case of the benzhydryl group, the p-methoxybenzyl group etc., or by contacting with water in the case of the trimethylsilyl group.

Further, the removal of a protective group for the amino group may be easily conducted by hydrolysis where it is an aralkyl group such as trityl or one of the various acyl groups. As the acid used on this occasion, formic acid, trifluoroacetic acid, hydrochloric acid etc. are preferred.

Furthermore, it is also possible to simultaneously conduct the removal of protective groups for the carboxyl and amino groups. As the reaction solvent, methanol, acetone, dichloromethane, tetrahydrofuran, dimethylsulfoxide, dimethylformamide etc. may be used either singly or in appropriate mixture.

It is then possible to esterify the carboxyl group at the 3-position of the cephalosporin compound ($I_1$) using halogen compound (IV). The carboxyl group in the formula ($I_1$) compound may be free (free carboxylic acid) or in the form of a reactive derivative such as a salt. As halogen compound (IV), for example, 1-iodoethyl acetate, 1-bromoethyl acetate, 1-iodoethyl 1-cyclohexyl acetate, 1-bromoethyl 1-cyclohexyl acetate, iodopivaloyloxymethyl, chloropivaloyloxymethyl etc. may be used.

The esterification reaction can be conducted in an appropriate solvent using halogen compound (IV) in an equimolar or excess amount relative to compound ($I_1$). As the solvent, dimethylformamide, acetone, dimethylsulfoxide etc. are suitable. The reaction may proceed easily at room temperature or with cooling, but in order to promote the reaction, a base such as potassium or sodium bicarbonate etc. may be added.

Thereafter, any protecting groups in the product can be removed as described above

The salts of compounds (I) may be produced by using a salt of the starting material in an above-described process, or by applying a conventional salt-forming reaction to the initially formed free compound.

Isolation and purification of the compounds of the present invention may be conducted in conventional manner; separation and purification by extraction with organic solvents, crystallization, column chromatography etc. may be employed.

## Examples

Compounds of the present invention and their production are illustrated in more detail in the following Examples.

### Example 1

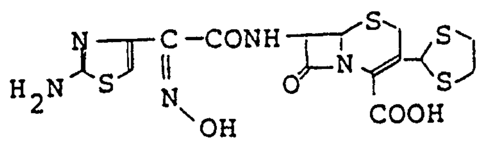

(1) 5.39 g of (Z)-2-hydroxyimino-2-(2-tritylamino-4-thiazolyl)acetic acid is suspended in 105 ml of dichloromethane, and 3.6 ml of 2-methoxypropene is added at 10° C. After stirring at room temperature for 30 minutes, the solvent is distilled off under reduced pressure; 75 ml of dichloromethane is added to the residue, 2.74 g of phosphorus pentachloride is added at -25° C, and stirred at -20 to -15° C for 30 minutes to obtain a solution of (Z)-2-(1-methoxy-1-methyl)ethoxyimino-2-(2-tritylamino-4-thiazolyl)acetic acid chloride.

3.50 g of trifluoroacetic acid salt of 7-amino-3-(1,3-dithiolan-2-yl)-3-cephem-4-carboxylic acid is suspended in 70 ml of dichloromethane, and 4.13 ml of bis(trimethylsilyl]acetamide is added thereto at 10° C. After stirring at room temperature for 15 minutes, 3.38 ml of pyridine and the above-described acetic acid chloride solution are added successively at -65° C. After stirring at -40 to 35° C for 30 minutes, the mix is poured into 350 ml of a saturated aqueous solution of monopotassium phosphate, and extracted with 100 ml of dichloromethane twice. The organic layer is washed with 50 ml of a saturated solution of monopotassium phosphate, and dried on anhydrous magnesium sulfate. The residue obtained by distilling off the solvent under reduced pressure was subjected to silica gel column chromatography with chloroform - isopropanol - formic acid (100:3:0.3) to obtain 2.63 g (yield 40%) of (Z]-3-(1,3-dithiolan-2-yl)-7-[2-(1-methoxy-1-methyl]-ethoxyimino-2-(2-tritylamino-4-thiazolyl)acetamido]-3-cephem-4-carboxylic acid.

IR Spectrum $\nu_{max}^{KBr}$ $cm^{-1}$; 3415, 2980, 2945, 1790, 1685, 1530, 1070, 700

NMR Spectrum (DMSO-$d_6$) $\delta$(ppm];

1.19 (6H, s, $CH_3$ x 2),

3.12 (3H, s, $OCH_3$),

3.18 - 3.48 (4H, m, 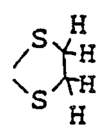 ),

3.69 (2H, s, 2-$CH_2$),

5.18 (1H, d, 6-CH),

5.68 (1H, dd, 7-CH),

5.99 (1H, s, 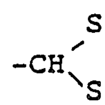 ),

6.72 (1H, s, 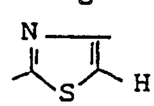 ),

7.16 - 7.52 (15H, m, $C\varnothing_3$),

7.74 (1H, br, s, NH),

9.47 (1H, d, CONH).

Positive Ion - FAB - Mass Spectrum; m/z

788 $(M+H)^+$

(2) 9.01 g of (Z)-3-(1,3-dithiolan-2-yl)-7-[2-(1-methoxy-1-methyl)ethoxyimino-2-(2-tritylamino-4-thiazolyl)-acetamido]-3-cephem-4-carboxylic acid is dissolved in 65 ml of dichloromethane, and 260 ml of 80% acetic acid is added. After stirring at 35 -40° C for an hour, the solvent is distilled off under reduced pressure. The residue is azeotropically distilled (twice) with 200 ml of ethanol, and 485 ml of ether is added to make it a powder. The powder is recovered by filtration and dried to obtain 1.31 g (yield 92%) of (Z)-7-[2-(2-amino-4-thiazolyl)-2-(hydroxyimino)acetamido]-3-(1,3-dithiolan-2-yl]3-cephem-4-carboxylic acid.

IR Spectrum $\nu_{max}^{KBr}$ $cm^{-1}$; 3450, 1770, 1670, 1635, 1535.

NMR Spectrum (DMSO-$d_6$) $\delta$ (ppm);

3.20 - 3.57 (4H, m, 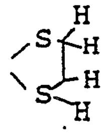 ),

3.70 (2H, s, 2-$CH_2$),
5.20 (1H, d, 6-CH),
5.75 (1H, dd, 7-CH),

6.01 (1H, s, 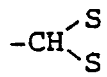 ),

6.68 (1H, s, 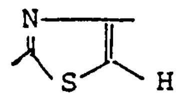 ),

7.12 (2H, br, s, $NH_2$),
9.45 (1H, d, CONH).
Positive Ion - FAB - Mass SPectrum; m/z
474 $(M+H)^+$

Example 2

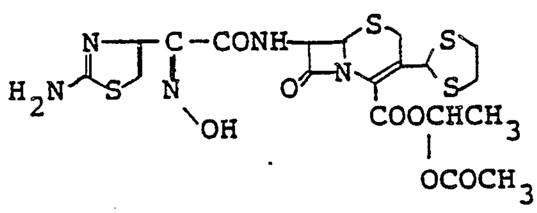

616 mg of (z)-[7-(2-amino-4-thiazolyl]-2-(hydroxyimino)-acetamido]-3-(1,3-dithiolan-2-yl)-3-cephem-4-carboxylic acid is dissolved in 20 ml of dimethylformamide, then 100 mg of potassium carbonate is added, and thereafter 2ml of a solution of 306 mg of 1-iodoethyl acetate in dimethylformamide is added. After stirring at -10 to 2°C for 20 hours, the solvent is distilled off under reduced pressure, and 20 ml of ether is added to the residue to make it a powder. The powder is recovered by filtration, and subjected to silica gel column chromatography with chloroform - methanol - formic acid (90:10:2) to obtain 184 mg (yield 25%) of 1-acetoxyethyl (Z)-7-[2-(2-amino-4-thiazolyl)-2-(hydroxyimino)acetamido]-3-(1,3-dithiolan-2-yl)-3-cephem-4-carboxylate.
IR Spectrum $\nu_{max}^{KBr}$ $cm^{-1}$; 3360, 2940, 1780, 1675, 1620, 1535, 1380, 1210, 1075, 1000, 945, 865.
NMR Spectrum (DMSO-$d_6$) $\delta$ (ppm);
1.46 (3H, dd, $CH_3$),
2.06 (3H, s, $CH_3CO$),

3.15 - 3.46 (4H, m, 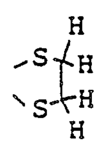 ),

3.70 (2H, s, 2-$CH_2$),
5.20 (1H, dd, 6-CH),

5.63 - 5.90 (2H, m, 7-CH, 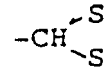 ),

6.63 (1H, s, 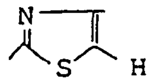 ),

6.90 (1H, m, 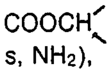 ),
7.09 (2H, br, s, $NH_2$),
9.43 (1H, d, CONH).
Positive Ion - FAB - Mass Spectrum; m/z
560 $(M+H)^+$

Example 3

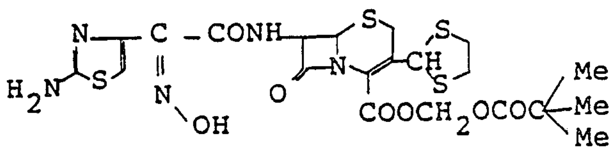

In a manner similar to that in Example 2, 113 mg of (Z)-7-[2-(2-amino-4-thiazolyl)-2-(hydroxyimino)-acetamido]-3-(1,3-dithiolan-2-yl)-3-cephem-4-carboxylic acid is dissolved in 5 ml of dimethylformamide, and treated by adding 18 mg of potassium carbonate, to obtain 18 mg (yield 12.9%) of 1-pivaroyloxymethyl (Z)-7-[2-(2-amino-4-thiazolyl)-2-(hydroxyimino)acetamido]-3-(1,3-dithiolan-2-yl)-3-cephem-4-carboxylate.

IR Spectrum $\nu_{max}^{KBr}$ $cm^{-1}$; 3368, 2984, 2940, 1790, 1754, 1678, 1618, 1530, 1372, 1278, 1122, 992
NMR Spectrum (DMSO-$d_6$) $\delta$ (ppm);
1.20 (3H, s, $CH_3$),

3.50 - 3.52 (4H, m, 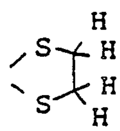 ),

3.77 (2H, dd, 2-$CH_3$),
5.26 (1H, d, 6-CH),

5.81 - 5.84 (3H, m, $-COOCH_2-$, 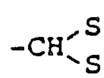 ),

5.95 (1H, q, 7.-CH),

6.69 (1H, s, 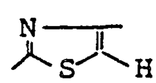 ),

9.46 (1H, d, -CONH-).
Positive Ion - FAB - Mass Spectrum; m/z
588 $(M+H)^+$; 610 $(M+Na)^+$

**Claims**

1. A 7-[2-(2-amino-4-thiazolyl)-2-(hydroxyimino)-acetamido]-3-(1,3-dithiolan-2-yl]-3-cephem-4-carboxylic acid derivative of formula I or a salt thereof

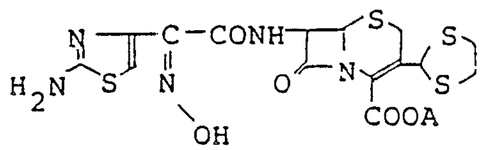

(I)

wherein A represents a hydrogen atom, a 1-acetoxyethyl group, a 1-cyclohexylacetoxyethyl group or a pivaloyloxymethyl group.

2. A compound according to claim (1) which is (2)-7-[2-(2-amino-4-thiazolyl]-2-(hydroxyimino]-acetamido]-3-(1,3-dithiolan-2-yl)-3-cephem-4-caboxylic acid.

3. A compound according to claim (1) which is 1-acetoxyethyl (Z)-7-[2-(2-amino-4-thiazolyl)-2-(hydroxyimino)acetamido]-3-(1,3-dithiolan-2-yl]-3-cephem-4-carboxylate.

4. A compound according to claim (1) which is 1-pivaroyloxymethyl (Z)-7-[2-(2-amino-4-thiazolyl]-2-(hydroxyimino)acetamido]-3-(1,3-dithiolan-2-yl)-3-cephem-4-carboxylate.

5. A compound according to claim (1) which is 1-cylcohexylacetoxyethyl (Z)-7-[2-(2-amino-4-thiazolyl)-2-(hydroxyimino)acetamido]-3-(1,3-dithiolan-2-yl]-3-cephem-4-carboxylate.

6. A method of making a compound of formula I or a salt thereof:

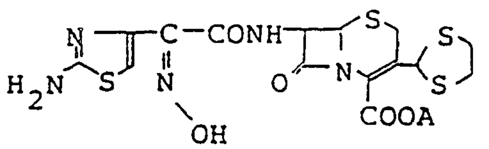

(I)

wherein A represents a hydrogen atom, a 1-acetoxyethyl group, a 1-cyclohexylacetoxyethyl group or a pivaloyloxymethyl group, which comprises (a) reacting compound II or salt thereof with compound III or salt thereof to form compound $1_1$ or salt thereof and optionally (b) reacting the latter with compound IV to form compound $1_2$, as follows:

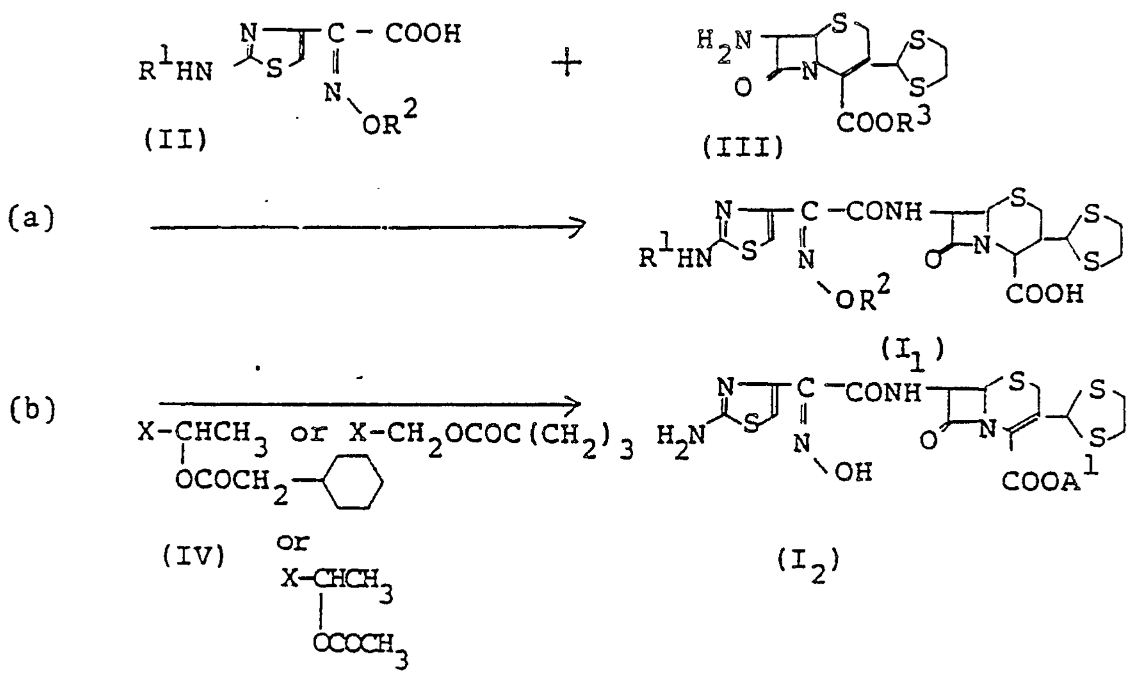


(II) (III) ($I_1$) (IV) ($I_2$)

wherein $A^1$ represents a 1-acetoxyethyl group, a 1-cyclohexylacetoxyethyl group or a pivaloyloxymethyl group, $R^1$ represents a hydrogen atom or a protective group for the amino group, $R^2$ represents a hydrogen atom or a protective group for the hydroxyl group, $R^3$ represents a hydrogen atom or a protective group for the carboxyl group, and X represents a halogen atom, any protective group(s) being removed from the desired product, and product $I_1$ and/or $I_2$ optionally being converted to or from salt form.

7. An antimicrobial composition for oral administration including a compound according to any of claims 1 to 6.